Europäisches Patentamt

European Patent Office

Office européen des brevets

(11) Publication number: **0 398 322 A1**

# EUROPEAN PATENT APPLICATION

(21) Application number: **90109327.8**

(22) Date of filing: **17.05.90**

(51) Int. Cl.⁵: **C07K 7/08, C07K 7/10, A61K 37/02, A61K 39/00**

(30) Priority: **19.05.89 IT 2055489**

(43) Date of publication of application:
**22.11.90 Bulletin 90/47**

(84) Designated Contracting States:
**AT BE CH DE DK ES FR GB GR LI LU NL SE**

(71) Applicant: **SCLAVO S.p.A.**
**Via Fiorentina 1**
**I-53100 Siena(IT)**

(72) Inventor: **De Magistris, Maria Teresa**
**Via S. Marco, 115**
**I-53100 Siena(IT)**
Inventor: **Bartoloni, Antonella**
**Via Vignali, 16, Località Costalpino**
**I-53100 Siena(IT)**
Inventor: **Rappuoli, Rino**
**Via Calamandrei, 39**
**I-53035 Quercegrossa/Monteriggioni(IT)**
Inventor: **Tagliabue, Aldo**
**Chiesa S. Leonardo, Catignano, Pianella**
**I-53019 Castelnuovo Berardenga, Siena(IT)**

(74) Representative: **Gervasi, Gemma et al**
**NOTARBARTOLO & GERVASI Srl Viale**
**Bianca Maria 33**
**I-20122 Milan(IT)**

(54) **Synthetic peptides capable of stimulating a T-cellular immunity.**

(57) The S1 subunit regions of pertussis toxin from Bordetella pertussis comprising the antigenic sites recognized by the human T helper clones anti-S1 are described and said sites are characterized.

Synthetic peptides having an amino acid sequence corresponding to the sequence of said regions or of the T-epitopes comprised therein are novel.

Said peptides, alone or in combination with the immunodominant B-epitope of the pertussis toxin S1 subunit from Bordetella pertussis, are particularly useful as immunogens for manufacturing acellular anti-pertussis vaccines.

## SYNTHETIC PEPTIDES CAPABLE OF STIMULATING A T-CELLULAR IMMUNITY

The present invention generally relates to an anti-pertussis vaccine.

The present invention specifically relates to the localisation of the pertussis toxin (PT) S1 subunit region comprising the epitopes recognized by the anti-S1 and anti PT T-helper lymphocytes and the characterisation of said epitopes.

More specifically the present invention relates to synthetic peptides having an amino acid sequence corresponding to the sequence of said regions or of the epitopes therein comprised able to elicit a T-cellular immunity anti-pertussis toxin.

The present invention still relates to the use of said peptides alone or in combination with the immunodominant B epitope of the PT S1 subunit, as immunogens for manufacturing acellular anti-pertussis vaccines capable of eliciting a protective immunity against this infection.

Pertussis is a disease of the respiratory tract caused by Bordetella pertussis ( B. pertussis ) a bacillus which is transmitted through the air from a sick person to a healthy sensitive person during the catarrhal convulsive phase.

Since this disease may cause, specifically by children and by babies lacking maternal anti-PT antibodies, convulsions, cerebral injuries and death, a vaccine effective for preventing this infection is particularly desirable.

The first anti-pertussis vaccines, consisting of inactivated B. pertussis cells (cellular vaccines) were produced some 40 years ago and have been successfully used many years.

However in the last decade the use of the cellular vaccine was dramatically reduced due to the side effects like swellings, fever, convulsions and sometime shock and death associated with it and with following break through of pertussis occurrence.

For this reason the interest in searching new antigens protective against B. pertussis suitable for manufacturing acellular vaccines free of the above cited drawbacks has been growing in the last years.

The pertussis toxin (PT), the filamentous haemoagglutinin (FHA) and a 69000 dalton membrane protein were proposed among other candidates for developing such vaccines, since a T cellular memory against those antigens was observed in immune donors.

Of particular interest is the pertussis toxin since highly effective in protecting in animal models.

The recent cloning of the genes encoding the five subunits ( S1, S2, S3, S4 and S5) of the PT ( Italian Patent Application No. 19208 A/86 and 21314 A/86) provides the possibility of developing acellular anti-pertussis vaccines based essentially on the use of recombinant PT antigen.

Until present however, the efforts to obtain the expression of the PT complete of its subunits by employing B. subtilis have been unsuccessful. Moreover the use of E. coli as a host organism did not enable the production of the PT in natural form.

The interest was therefore focused on the enzymatically active PT subunit as a substitute of the pertussis toxin in an acellular anti-pertussis vaccine.

In fact the use of monoclonal and polyclonal antibodies anti-PT had proved that the S1 was the PT subunit exhibiting the highest immunogenity ( Sato H. et al. 1984, Infect. Immun. 46:422; Bigio M. et al. 1988 FEMS Microbiol. Letters, 51:7). Moreover monoclonal antibodies anti- S1 were observed to neutralize in vitro the pertussis toxin ( Bigio M. et al.1988 FEMS Microbiol. Letters 51:7) and to induce an in vivo protection (mice) effective against the virulent B. pertussis intracerebral infection (Sato H. et al. 1984, Infect. Immun. 46:422).

However, the recombinant S1, obtained as N-terminal fused protein with the 98 amino acid sequence of E. coli MS2 fage polymerase, as reported in the above quoted Italian applications, is ineffective in eliciting in animal models protective antibodies against pertussis, likely because of conformational problems.

Therefore the recombinant antigens PT and S1 would appear unsuitable candidates for preparing effective acellular anti-pertussis vaccines.

For this reason the researchers' interest was focused on the production of synthetic vaccines comprising no longer the complete antigens, but peptides reproducing small regions of the antigen, known as epitopes or antigenic determinants or sites and capable of inducing the production of specific antibodies able to neutralize the infecting agent.

More specifically the development of acellular anti-PT vaccines based on the use of such peptides needs the identification of the antigenic determinants for the T lymphocytes which, together with the B protective antigenic sites, induce a specific immune-response against the PT protein.

Recently the characterisation of the B protective epitope of the pertussis toxin S1 and the synthesis of peptides exhibiting a corresponding amino acid sequence were disclosed in the Italian patent application No. 23085 A/87.

So far however nothing was reported about the T epitope or epitopes of said antigen.

It is therefore an object of the present application localizing and identifying the pertussis toxin S1 antigenic sites recognized by the T lymphocytes specific for the native PT and S1 protein.

Still an object of the present invention are synthetic peptides having an amino acid sequence corresponding to the sequence of such sites capable of eliciting a T cellular immunity against pertussis.

A further object of the present invention is the use of said synthetic peptides as immunogens for manufacturing anti pertussis vaccines capable of eliciting in humans a protective immuno response specific for pertussis toxin.

Still further objects of the present invention will be evident in the light of the description and examples which follow.

According to the present invention localising of the S1 antigenic sites recognized by the T lymphocytes has been carried out by making use of human T cellular clones specific for the PT and S1.

Peripheral blood lymphocytes of a donor, who contracted in his childhood pertussis, were found, in a previous study, to be capable of giving a high proliferative response in presence of inactivated B. pertussis.

According to the present invention, the same donor has been employed for generating T clones specific for the PT and for the S1 subunit.

In the practice the peripheral blood mononuclear cells ( PBMC), isolated from the heparinized blood of said donor, have been restimulated in vitro by growing them at 37° C for a week on suitable medium such as e.g. RPMI 1640 added with human serum, in presence of varying concentrations of the heat-inactivated pertussis toxin and of the recombinant purified S1 subunit.

Thereafter the culture medium has been added with recombinant interleukin 2 (rIL-2) in order to expand the lymphocytes specific for PT and S1.

After 7 days at 37° C lymphocytes have been cloned. For this purpose different methods may be applied known as micromanipulation, agar cloning or limited dilutions.

The limited dilution technique was preferably employed, which contemplates growing a few cells, generally 0.3 cells per well, in RPMI in presence of phytohaemagglutinin, rIL-2 and allogenic PBMC cells treated with mitomicin C at 37° C for 7-14 days.

This is a critical phase of the process, since the cells are not able to survive if the conditions are not the optimal ones.

Thereafter the positive clones were selected making use of the in vitro proliferation assay in presence of the antigens PT and recombinant S1.

Such an assay was carried out as usual by incubating the T clones RPMI medium added with fetal calf serum (FCS) in presence of the above antigens and authologue B lymphocytes, i.e. lymphocytes belonging to the same donor organism, transformed by Epstein- Barr virus and treated with mitomicin C (EBV-B).

The cells EBV-B prepared according to Rosen A. et al. 1983 J. Immunol. 130:2899, are intended to present the antigens to the T lymphocytes.

All the cultures were pulse-labelled for at least 16-18 hours with [³H] thymidine and then the incorporated radioactivity was determined with a radioactivity counter.

The results of the proliferation assays were represented as the average of three counts per minute (cpm) of three identical cultures plus the standard deviation. Results were considered positive when SI > 3, where for SI is meant the ratio of the cpm of the cultures with and without antigen.

12 T clones specific for PT and 8 T clones specific for S1 were isolated following the above process (Table 1).

Said T clones, analysed through direct immunefluorescence on a fluorescence activated cell selector, making use of monoclonal antibodies OKT3, OKT4 and OKT8 conjugated to phicoerytrin or fluorescin, exhibited a phenotype helper / inducer CD3⁺, CD4⁺ CD8.

In order to identify the PT subunits recognized by each of the 12 clones, proliferation assays were carried out by contacting said clones with the recombinant subunits S1, S2, S3, S4, and S5 expressed as fused proteins in cells of transformed E. coli.

The obtained results (Table I) showed that 10 out of 12 T clones anti-PT (83%) were specific for S1 so confirming the immunodominat nature of this subunit over the others.

A clone was specific for the subunit S4 and an other clone for the subunits S2 and S3 which exhibit an homology of 67% indeed. No T clones proliferated in presence of the S5 subunit.

According to the present invention the S1 regions comprising the antigenic sites recognized by the 18 anti-S1 T clones previously isolated are localized and identified.

In practice in vitro proliferation assays were carried out making use of S1 protein fragments free of the N-terminal or C-terminal or both sequences of different length as antigens.

3

Said fragments were prepared by recombinant DNA as reported in the Italian patent application No. 23085 A/87 through expression of E. coli cells transformed with hybrid plasmids comprising the nucleotidic sequence coding for each of them.

Said sequence was obtained by digestion of the gene coding for the S1 comprised in the plasmid PTE 255 with the suitable restriction enzymes.

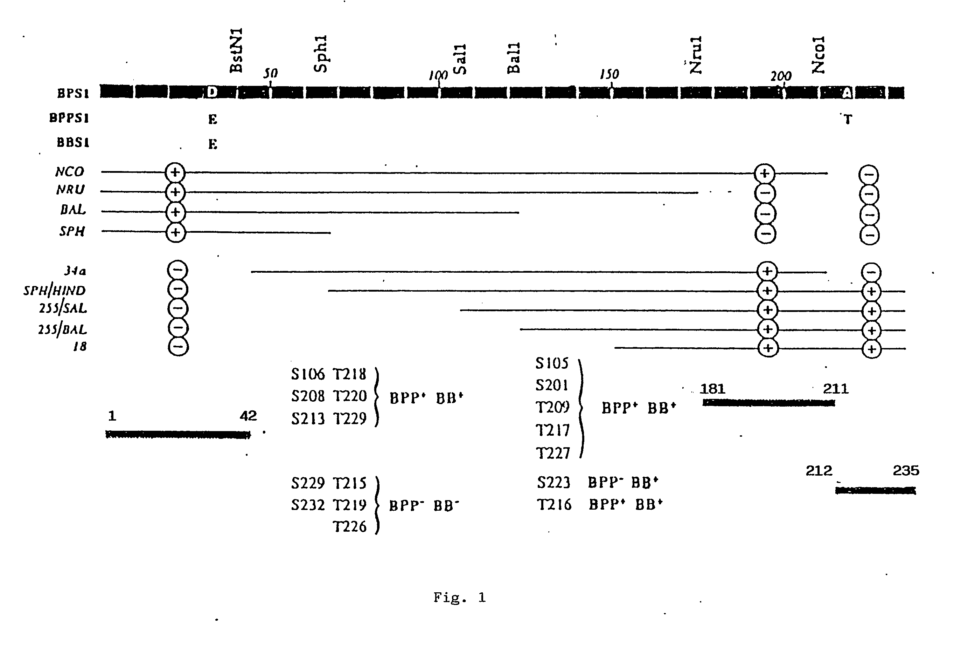

Specifically the following S1 peptic fragments were prepared: NCO (1-211) free of the C-terminal aminoacid sequence 212-235; NRU (1-180) free of the C-terminal aminoacid sequence 181-235; BAL (1-123) free of the C-terminal sequence 124-235; SHP (1-68) free of the C-terminal aminoacid sequence 69-235; 34a (43-211) free of the N-terminal 1-42 and C-terminal 212-235 aminoacid sequences ; SPH/HIND (67-235) free of the N-terminal aminoacid sequence 1-66; 255/SAL (109-235) free of the N-terminal aminoacid sequence 1-108; 255/BAL (124-235) free of the N-terminal aminoacid sequence 1-123; 18 (149-235) free of the N-terminal aminoacid sequence 1-148. The results of proliferation referred to in Table II and illustrated in figure 1 showed that 11 out of 18 examined T cellular clones mapped within the S1 N-terminal end.

In fact they recognized the NCO, NRU, BAL and SPH peptic fragments free of the S1 C-terminal aminoacid sequences, but they did not recognize fragments with deletions within the N-terminal part of the protein.

The shortest N-terminal deletion still abolishing the clone proliferation was that of the fragment 34a of 42 amino acids.

Therefore the epitope or epitopes recognized by said clones were likely comprised within the S1 aminoacid sequence 1-42, though it may also comprise some N-terminal residues of the 34a fragment.

On the contrary the other seven clones mapped within the S1 C-terminal end. In particular 5 of them recognized all fragments with deletions within the S1 N-terminal end comprising the 34a fragment free of the last 24 C-terminal aminoacid residues. Only the NCO which has a 24 aminoacid deletion as the 34a, among all C-terminal deletion fragments, was recognized by the 5 T clones.

Those results pointed out that the T epitope or epitopes recognized by said clones were contained within the region 181-211 of S1 comprised between the last NRU C-terminal residue and the last NCO C-terminal residue.

In any case such a region may result even longer and may comprise other C-terminal aminoacid residues of the NRU peptic fragment.

Finally, the last 2 anti-S1 T clones mapped within the S1 region 212-235.

Three S1 regions (1-42, 181-211 and 212-235) are thus identified, which comprise the epitopes recognized by the human T clones anti-S1.

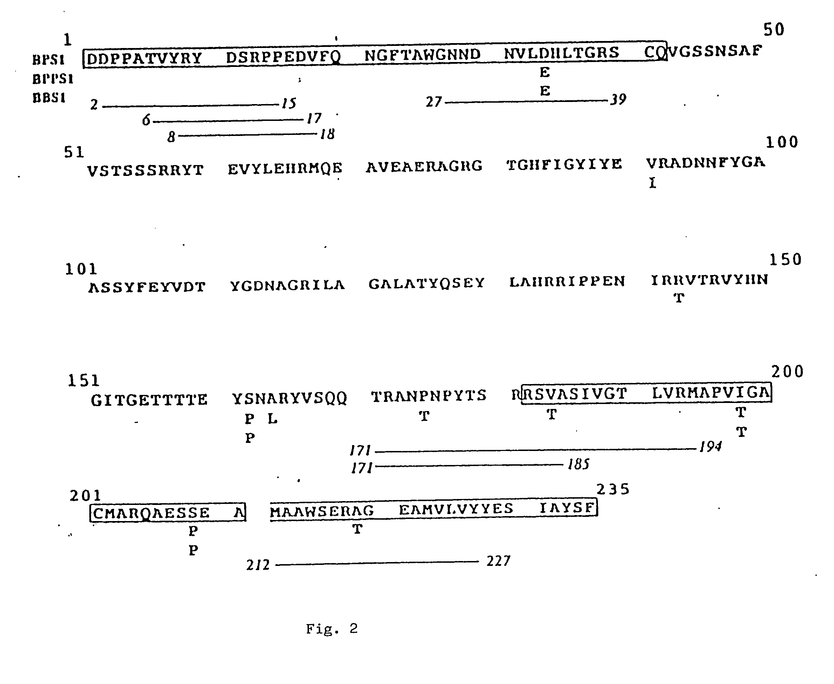

The sequences of the above regions are referred to in figure 2.

It is moreover interesting noting that the anti-S1 clones obtained by using either the purified PT or the recombinant S1 exhibit similar specificity, so proving that the fusion of the S1 with the MS2 fage polymerase does not affect the protein immunogenity.

According to the present invention and in order to better localize the T epitopes within the above S1 regions, the anti-S1 T clones were assayed by in vitro proliferation using as antigens the recombinant S1 subunits of B. parapertussis (BPPS1) and B. bronchiseptica (BBS1), two species correlated to B. pertussis which differ from its S1 subunit (BPS1) merely because of the substitution of a few amino acids.

In particular, some of these substitutions involve the regions recognized by the T clones. The obtained results showed in two cases that such substitutions completely abolished the clones proliferation thus pointing out that the substituted amino acids may be part of one epitope.

In the first case the mutation at issue was the replacement of Aspartic acid (Asp) at the position 34 with Glutamic acid (Glu) for both BBS1 and BPPS1 proteins.

In fact said substitution abolished the ability to recognize antigens of 5 T clones which mapped within the 1-42 region.

In the second case, the mutation at issue involved the substitution of the amino acid residue Alanin (Ala) at the position 219 with Threonine (Thr).

This mutation was indeed the reason of the lack of reactivity with BPPS1 of one of the two clones which mapped within the BPS1 212-235 region.

In order to identify within the S1 regions 1-42, 181-211 and 212-235 shorter amino acid sequences capable of stimulating the T cells, peptides have been synthesised which exhibit an amino acid sequence homologous to that of fragments of said regions. In particular, the following peptides have been synthesised:

1. DPPATVYRYDSRPP, corresponding to the S1 sequence 2-15;

2. TVYRYD SRPPED, corresponding to the S1 sequence 6-17.

3. YRYDSRPPEDV, corresponding to the S1 sequence 8-18;

4. GNNDNVLDHLTGR, corresponding to the S1 sequence 27-39;

5. TRANPNPYTSRRSVASIVGTLVRM, corresponding to the S1 sequence 171-194:

6. TRANPNPYTSRRSVA, corresponding to the S1 sequence 171-185;

7. MAAWSERAGEAMVLVY, corresponding to the S1 sequence 212-227.

The amino acids are shown with single letter codes where:

```
asp D   thr T   glu E   gly G   lys K   ala A   arg R   val V

his H   leu L   tyr Y   ile I   cys C   met M   asn N   pro P

gln Q   phe F   ser S   trp W.
```

The above peptides may be synthetised according to any of the known general techniques.

In particular they were synthesised according to the method described by Merrifield et al. ( Merrifield R.B. J.Am. Chem. Soc. 1983, 85, 2149-2154).

The peptides purified by chromatography were used as antigens in proliferation assays for stimulating anti-S1 T clones mapping within the N-terminal end of the S1 subunit (region 1-42).

The obtained results (see Table III) showed that three of five clones recognizing BPS1 but not BBS1 and BPPS1 proliferated in presence of the peptide 27-39 homologous to the sequence which comprises in BBS1 and BPPS1 the mutation Asp-Glu [34]. This proved the presence of an immunodominant antigenic site localized about the Asp residue and allowed to define said epitope as a sequence of 13 amino acid.

The absence of proliferation of the other two clones in presence of the 27-39 peptide suggested a likely polyclonal response directed against said antigenic site.

The six which mapped within the S1 N-terminal end and which were not affected by the Asp-Glu [34] mutation did proliferate in presence of the 2-15, 6-17, 8-18 synthetic peptides.

According to the present invention, the five clones mapping within the S1 C-terminal end (region 181-211) were assayed for the ability to recognize the 171-194 and 171-185 peptides.

These two synthetic peptides shared the first 15 amino acids, 10 of which were homologous to the C-terminal part of the NRU fragment.

As reported in Table III, four of the five T clones proliferated in presence of the 171-194 peptide but not in presence of the 171-185 peptide.

These results suggested that an epitope specific for said T clones might be comprised within the C-terminal second half of the 171-194 sequence.

None of the two clones mapping within the 212-235 region was stimulated by the 212-227 peptide.

The variability in specificity of clones mapping within the same region, pointed out also by using the BPPS1 and BBS1 proteins, may be construed as meaning that: either different epitopes are comprised in the same region or a polyclonal T cellular response is directed against a one unic immunodominant site.

In any case the behaviour of the two clones which were affected in recognizing the BPPS1 and BBS1 proteins by the Asp-Glu mutation at the position 34, but which were different from other similar clones because they were not stimulated by the 27-39 peptide, would appear to confirm the existence of a heterologous T cellular response against the immunodominant antigenic site located around the Asp[34] residue.

According to the present invention, the analysis of the primary sequence of the 27-39 peptide showed the presence of the residues Asn-Val-Leu-Asp (31-34) corresponding to the consensus sequence shared by most of T-cell determinants (Rothbard, J.B. and Taylor, W.R., EMBO J., 7: 93, 1988).

Moreover the residue 26-42 seemed to form an amphypatic helix, which feature is specifically associated with the antigenic T sites (Margalit, H. et al. J. Immunology 138: 2213, 1987).

In a graphic representation of this amphypatic helix, the residue 34 is located on the hydrophilic part, it is therefore possible to argue that the Asp[34] interacts with the T lymphocyte receptor when the antigen is being recognized. The same analysis applied to the 171-194 peptide disclosed that the sequence 181-198 showed a high degree of amphypaticity in agreement with the Margalit et al. algorithm.

This supported the hypothesis that an epitope is located within the second half of the sequence 171-194.

It is moreover interesting to observe that the 1-42 and 181-211 regions, which comprise the epitopes recognized by the most T clones analysed in the present application, also include part of the amino acid residues which take part of the sequence of the conformational B epitope recognized by the protective anti-S1 monoclonal antibodies.

Therefore synthetic peptides with an amino acid sequence corresponding to the sequence of the S1 1-42, 181-211, 212-235 regions, as identified according to the present invention, or to the sequence of the T epitopes comprised in said regions, are specifically useful either alone or associated with the S1 B epitope, for preparing acellular anti-pertussis vaccines.

Of course, pharmaceutic compositions as vaccines, comprising as active agent the peptides of the present invention in an amount sufficient to develop an immunogenic effect in a susceptible host and a pharmaceutically acceptable, non-toxic carrier, can easily be manufactured in the light of the disclosure in the present patent application.

Such carriers may be non-toxic buffer, saline solution or similar. The composition may be administered systemically and may be in any suitable form.

The peptides of the present invention may be administered combined to other antigens, adjuvants, protease inhibitors or any compatible substance, when such a combination is regarded as desirable or advantageous for controlling pertussis infection.

The T epitopes of the present invention, when used alone or linked to other antigenic sequences derived from PT, enable the production of an anti-pertussis vaccine exhibiting a broader activity and a longer efficacy and being free of the toxicity due to the complete PT.

Moreover the T epitopes of the present invention may be conjugated to other peptides or polysaccharides and may be used as carriers by preparing vaccines against other infections.

BRIEF DESCRIPTION OF THE DRAWINGS

Figure 1: Recombinant S1 fragments recognized by the T clones specific for S1. The clones designated as S were obtained by using recombinant S1, whereas those designated as T were obtained with purified PT. The sign (+) shows the recombinant fragments recognized by the clones mapping within the corresponding regions, illustrated by dashed segments at bottom of the figure.

Figure 2: The BPS1 amino acid sequences and the BPPS1 and BBS1 mutations are elucidated. The synthetic peptides homologous to BPS1 used to stimulate the T clones are also reported. The sequence put in evidence represent the regions 1-42, 181-211 and 212-235 which comprise the T epitopes.

The following examples are provided by way of illustration and not by way of limitation of the invention.

Example 1.

Isolation of the human T anti-PT and anti-S1 clones.

A) Preparation of the recombinant PT and S1, S2, S3, S4 and S5 antigens.

The pertussis toxin (PT) was purified from supernatant of B. pertussis culture during the phase I, as reported by Sekura, R.D. et al. (J. Biol. Chem., 258: 14647, 1983), precipitated with ammonium sulphate and maintained at 4° C.

Before being used, the PT was suspended in saline buffer (saline solution) and inactivated at 100° C for 45 minutes in order to eliminate its mitogenic effect.

The subunits S1, S2, S3, S4, and S5 were obtained as fused proteins i.e. as proteins comprising at the N-terminal region the 98 amino acid sequence from fage MS2 polymerase, by culturing E. coli cells transformed as disclosed in the Italian patent applications No. 19208 A/86 and 21314 A/86.

The so obtained proteins were partially purified by cellular lysis and following recovery of the enclosed bodies as described by Nicosia, A. et al. (Infect. Immun. 55:963 1987).

The resulting protein concentration was of 0.5-1 mg/ml, 50-70% of which consisting of the fused protein.

The enclosed bodies were resuspended in PBS and maintained at -20° C.

The recombinant S1 was further purified by electroelution as described by Nicosia, A. et al. 1987, Infect. Immunol. 55:963, before being used in the stimulation assay of the peripheral blood mononuclear cells (PBMC) of a donor immunized against pertussis.

B) Isolation of the T cellular clones.

Peripheral blood mononuclear cells were isolated from heparinized blood of a donor, who contracted pertussis in his childhood, making use of Ficoll-Hypaque (Pharmacia Fine Chemicals AB, Uppsala, Sweden).

Said cells were thereafter added to the wells of a 96 flat bottomed well plate, at the final concentration of $2 \times 10^5$ cells/well in 0.2 ml RPMI-HS medium (RPMI 1640 from GIBCO Laboratories, Paisley, Scotland), supplemented with L-glutamine (2 mM), 1% nonessential amino acids, 1% sodium pyruvate, 50 $\mu$g/ml gentamicin, $5 \times 10^{-5}$ M 2-mercaptoethanol and 10% heat inactivated human serum (HS).

The cells were grown in presence of different concentrations of inactivated PT and recombinant S1 (5 dilutions 1:3 starting from 10 $\mu$g/ml in the final volume) at the temperature of 37° C for 7 days. A control was carried out making use of the same concentrations of non-inactivated PT. A part of the cells was pulse-labelled with 1 $\mu$Ci[$^3$H] thymidine (sp. act. 185 GB q/mmole; Amersham Int., Amersham England) in order to prove the presence or absence of a cellular stimulation.

On day 7, all the wells were added with 50 U/ml recombinant interleukin-2 (Hoffmann La Roche, Nutley) to expand the proliferated cells.

After 7 further days at 37° C, the lymphoblasts were recovered from the wells and cloned by the method of limited dilutions according to Sinigaglia, F. et al. J. Immunol. 135:3929.

The cells were inoculated in amount of 0.3 cells per well on a Terasaki plate in presence of $10^4$ allogenic PBMC treated with mitomycin C, in 0.02 ml RPMI-HS containing 1 $\mu$g/ml phytohaemagglutinin (PHA) and rIL-2 (50 U/ml).

The clones were maintained in culture by periodic restimulation with allogenic PBMC treated with mitomycin C by the same conditions above reported.

C) Isolation of the T cellular anti-PT and anti-S1 clones by proliferation assay.

The T clones were added at the concentration of $2 \times 10^4$ cells/well, in each well of a 96 well plate containing 0.2 ml of RPMI medium supplemented with 10% fetal calf serum (FCS).

The clones were incubated at 37° C for 3 days in presence of 1 $\mu$g/ml of the antigens PT and recombinant S1 and of $2 \times 10^4$ mitomycin-C treated EBV-B cells (authologous lymphocytes B transformed with Epstein-Barr virus).

Said EBV-B cells are intended to supply the antigens thereby stimulating the proliferation of the T clones specific for said antigens.

All cultures were pulse-labelled with 1 $\mu$Ci [$^3$H] thymidine for 16-18 hours. The cells were then collected on glass fibre filter with a cell collector (Skatron, Lier, Norway). The radioactivity incorporated in the cells was counted with a scintillation counter as the number of cells incorporating radioactivity.

The results of the proliferation assays are reported as the average of the counts per minute (cpm) of a culture in triplicate plus/minus standard deviation.

The responses are regarded as positive for SI > 3, where SI means the ratio: cpm cultures with antigen / cpm cultures without antigen. Following the above conditions, 12 clones specific for PT (T106, T207, T209, T215, T216, T217, T218, T219, T220, T226, T227 and T229) and 8 specific for S1 (S105, S106, S201, S208, S213, S223, S229 and S232) were isolated (Table I).

In order to characterize the cellular surface phenotype of such clones, they were analysed by immunefluorescence with a FAC-star equipment, (Beeton, Dickinson, Erembdegen, Belgium) with monoclonal antibodies OKT3, 0KT4 and OKT8 conjugated either to ficoerytrin or to fluorescein (Ortho Diagnostic System, Raritan, NJ).

All the 20 clones exhibited the helper/inducer CD3$^+$, CD4$^+$ and CD8$^-$ phenotype.

D) Analysis of the specificity of the anti-PT T clones.

In order to identify the PT subunits recognized by each of the 12 T clones, proliferation assays were carried out, by using as antigens the single subunits S1, S2, S3, S4 and S5 obtained as reported in A) and operating as described in the previous step.

The obtained results (Table I) showed that 10 out of 12 T clones anti-PT were specific for S1, therewith confirming the immunodominance of said subunit over the others.

Among the other two clones, the former (T106) was specific for S4 the later (T207) recognized S2 and S3, which subunits have an homology of 67%.
None of the T clones recognized the subunit S5.

TABLE I

Proliferation of the T clones anti-PT and anti recombinat subunits

Clones      Antigens

| Clones | PT | S1 | S2 | S3 | S4 | S5 |
|---|---|---|---|---|---|---|
| **Clones T anti-PT** | | | | | | |
| RR-T106 | 2.9±0.3[a] | 5.1±0.6 | 2.2±0.2 | 2.2±0.0 | 2.4±2.7 | 9.4±1.4 | 2.6±0.2 |
| RR-T207 | 2.3±0.4 | 7.7±1.3 | 1.9±0.1 | 41.6±3.5 | 39.4±0.4 | 2.1±0.1 | 2.5±0.3 |
| RR-T209 | 2.4±0.5 | 5.8±0.5 | 18.7±1.7 | 2.2±0.0 | 2.4±0.0 | 2.4±0.2 | 2.6±0.3 |
| RR-T215 | 2.3±0.2 | 55.8±2.4 | 59.4±1.6 | ND | 2.7±0.3 | 2.1±0.1 | 2.2±0.2 |
| RR-T216 | 2.3±0.3 | 5.8±0.8 | 14.6±0.2 | 2.2±0.2 | 2.5±0.3 | 2.5±0.1 | 1.9±0.2 |
| RR-T217 | 2.0±0.7 | 4.7±0.8 | 16.1±2.3 | 1.3±0.1 | 1.9±0.3 | 1.6±0.1 | 1.4±0.1 |
| RR-T218 | 2.1±0.2 | 11.3±0.6 | 20.5±2.6 | 3.2±0.4 | 2.5±0.0 | 2.1±0.0 | 2.1±0.3 |
| RR-T219 | 2.6±0.2 | 9.9±1.7 | 10.4±0.4 | 2.2±0.0 | 2.1±0.1 | 1.9±0.0 | 1.8±0.0 |
| RR-T220 | 1.2±0.1 | 14.2±1.7 | 18.9±0.6 | 1.7±0.1 | 1.5±0.0 | 1.3±0.2 | 1.4±0.2 |
| RR-T226 | 2.4±0.2 | 57.3±2.4 | 56.0±2.2 | 3.2±0.8 | 2.3±0.2 | 2.1±0.1 | 2.2±0.0 |
| RR-T227 | 2.8±0.2 | 12.0±2.2 | 12.7±2.1 | 2.0±0.1 | 2.5±0.0 | 2.4±0.2 | 2.4±0.1 |
| RR-T229 | 2.5±0.1 | 32.6±1.6 | 31.7±1.3 | 4.6±0.8 | 2.4±0.1 | 2.5±0.2 | 2.3±0.4 |
| **Clones T anti S1** | | | | | | |
| RR-S105 | 2.2±0.3 | 12.8±2.3 | | | | | |
| RR-S106 | 2.7±0.2 | 63.5±2.8 | | | | | |
| RR-S201 | 0.7±0.1 | 13.4±1.0 | | | | | |
| RR-S208 | 2.5±0.2 | 29.3±1.0 | | | | | |
| RR-S213 | 0.9±0.1 | 46.0±4.8 | | | | | |
| RR-S223 | 2.5±0.3 | 15.3±1.4 | | | | | |
| RR-S229 | 2.9±0.4 | 9.0±0.3 | | | | | |
| RR-S232 | 3.0±0.5 | 10.6±0.8 | | | | | |

a = cpm average (x $10^{-3}$)± SD of three   cultures; the values in the boxes are statistically relevant
ND= not determined

Example 2.

Mapping the T epitopes of S1.

In order to identify the S1 regions comprising the epitopes recognized by the anti-S1 T clones, a series of proliferation assays was carried out, using as antigens nine recombinant S1 fragments of different length free either of the N-terminal sequence or of the C-terminal sequence or of both.

In practice said fragments were obtained by digestion of the plasmid PTE 255, which contains the gene encoding the S1, with the restriction enzymes Nco1, Nru1, Bal1, Sal1, Sph1 and BstN1, which cleave the gene at unique restriction sites, and operating as reported in the co-pending Italian patent application No. 23085 A/87.

The obtained recombinant fragments are specifically (figure 1): NCO (1-211) free of the C-terminal 212-235 sequence; NRU (1-180) free of the C-terminal 181-235 sequence; BAL (1-123) free of the C-terminal 124-235 sequence; SPH (1-68) free of the C-terminal 69-235 sequence; 34a (43-211) free of the C-terminal 212-235 and N-terminal 1-42 sequences; SPH/HIND (67-235) free of the N-terminal 1-66 sequence; 255/SAL (109-235) free of the N-terminal 1-108 sequence; 255/BAL (124-235) free of the N-terminal 1-123 sequence; 18 (149-235) free of the N-terminal 1-148 sequence.

The results illustrated in figure 1 showed that 11 of the anti-S1 T clones mapped within the N-terminal region of the S1 subunit.

In fact they recognized the fragments NCO, NRU, BAL and SPH but not fragments with N-terminal deletions. Since the shortest N-terminal deletion, still abolishing the clones proliferation, was that of the 34a fragment, it could be argued that at least one T epitope was comprised within the N-terminal 1-42 region. The remaining 7 clones (S105, S201, T209, T217, T227, S223 and T216) mapped within the C-terminal region of S1.

Among these clones, 5 (S105, S201, T209, T217 and T227) recognized all the N-terminal deleted fragments, comprising the 34a-fragment free of the C-terminal 212-235 sequence of 24 amino acids and the NCO fragment with the C-terminal 212-235 deletion.

The shortest C-terminal deletion, still abolishing the proliferation of the 5 clones, was that of NRU.

Thus these results proved that one or more T epitopes were contained within the 181-211 region of 31 amino acids, comprised between the C-terminal amino acid residue of NRU and that of NCO.

In any case this region could be longer and could comprise other amino acid residues of NRU.

Finally the clones S223 and T218 mapped within the S1 C-terminal 212-235 region of 24 amino acids. In fact said clones did not recognize the 34a and NCO fragments. On the basis of the above disclosure, it was possible to identify the following S1 regions comprising the T antigenic sites: 1-42, 181-211, 212-235.

For better localising of the T epitopes within said regions, proliferation assays of the T clones were carried out making use of the recombinant S1 subunits of B. parapertussis (BPPS1) and B. bronchiseptica - (BBS1) as antigens, which subunits differ from the S1 from B. pertussis (BPS1) because of the substitution of a few amino acids (figure 2).

The obtained results showed that, among the 11 clones mapping within the S1 N-terminal end, 5 (S299, S232, T215, T219 and T226) were not capable of recognizing BPPS1 and BBS1, suggesting that at least two different specificities about the T lymphocyte receptors existed in this group and enabling a better localisation of the epitope recognized by the 5 clones.

In fact the difference in the 1-42 sequences of BPS1 and BBS1 and BPPS1 was the mutation Asp-Glu at the position 34 (figure 2).

Since such a substitution is crucial for recognizing the protein, it is possible to assume that the amino acid Glu$^{34}$ takes part to the epitope recognized by the 5 clones. Finally, among the clones S223 and T216, mapping within the 212-235 region of BPS1, the former did not recognize BPPS1 showing in this region the mutation Ala-Thr at position 219.

EP 0 398 322 A1

TABLE II

Recognition of the mutated S1 and of the deleted fragments

Clones                                     Antigens

| | | BPS1 | BPPS1 | BBS1 | NCO | NRU | BAL | SPH | 34a | SPH-HIND | 255SAL | 255BAL | 18 |
|---|---|---|---|---|---|---|---|---|---|---|---|---|---|
| RR-S106 | 2.7[a] | 63.5 | 99.8 | 97.6 | 85.5 | 110.7 | 110.8 | 94.9 | 2.6 | 2.4 | 3.1 | ND. | 2.9 |
| RR-T229 | 1.4 | 9.5 | 8.9 | 11.3 | 9.1 | 16.4 | 8.1 | 11.0 | 1.5 | 1.1 | 1.1 | 2.3 | 1.3 |
| RR-S229 | 2.9 | 9.0 | 2.3 | 3.2 | 10.0 | 13.0 | 9.0 | 10.7 | 2.2 | 2.1 | 2.5 | 4.4 | 2.4 |
| RR-T219 | 1.1 | 17.7 | 0.8 | 3.1 | 20.2 | 27.1 | 20.5 | 38.5 | 0.8 | 0.8 | 0.7 | 1.7 | 1.0 |
| RR-T226 | 2.2 | 8.6 | 2.5 | 3.2 | 9.4 | 9.0 | 9.3 | 13.2 | 2.1 | 1.6 | 1.9 | 3.3 | 2.4 |
| RR-S105 | 2.2 | 12.8 | 4.7 | 33.3 | 33.9 | 2.5 | 2.0 | 2.4 | 16.4 | 52.0 | 58.9 | 55.3 | 46.0 |
| RR-T209 | 1.2 | 13.6 | 13.4 | 24.0 | 34.9 | 1.4 | 1.2 | 1.2 | ND | 118.3 | 98.0 | 63.0 | 53.3 |
| RR-T227 | 1.2 | 12.6 | 24.1 | 36.3 | 17.8 | 1.2 | 0.8 | 0.9 | ND | 51.8 | 44.1 | 26.2 | 16.4 |
| RR-S223 | 2.5 | 15.3 | 2.7 | 24.5 | 2.6 | 1.7 | 2.9 | 2.9 | 2.0 | 56.3 | 55.0 | 39.8 | 32.7 |
| RR-T216 | 1.7 | 16.5 | 19.9 | 25.8 | 2.4 | 2.5 | 1.6 | 3.4 | 1.6 | 33.1 | 37.8 | 24.3 | 21.9 |

a=cpm average(x $10^{-3}$) of three cultures
ND= not determined

Example 3

Recognition of the synthetic peptides.

In order to identify within the regions 1-42 and 181-211 the shortest amino acid sequence capable of stimulating the T cells, peptides having sequences homologous to fragments of said regions were synthetized.

Practically the following peptides were synthesised according to the method of Marrifield, R.B. ( J. Am. Chem. Soc. 1963, 85:2149-2154):

1. DPPATVYRYDSRPP corresponding to the 2-15 sequence of S1;
2. T VYRYDSRPPED corresponding to the 6-17 sequence of S1;
3. YRYDSRPPEDV corresponding to the 8-18 sequence of S1;
4. GNNDNVLPHLTGR corresponding to the 27-39 sequence of S1;
5. TRANPNPYTSRRSVASIVGTLVRM corresponding to the 171-194 sequence of S1;
6. T R ANPNPYTSRRSVA corresponding to the 171-185 sequence of S1;
7. M AAWSERAGEAMVLVY corresponding to the 212-227 sequence of S1.

The peptide purity was confirmed by amino acids analysis and HPLC. Said peptides were then used as T cells stimulants in proliferation assays.

The clones mapping within the N-terminal region were stimulated by the peptides 2-15, 6-17, 8-18 and 27-39.

As evident from Table III, three of the 5 clones capable of recognizing BPS1 but not BBS1 and BPPS1 were stimulated by the peptide 27-39. Therefore the epitope recognized by said three clones (S232, T215 and T226) was defined as a sequence of 13 amino acids corresponding to the peptide 27-39.

The clones S229 and T129, which were not stimulated by the peptide 27-39 and by the other peptides 2-15, 6-17 and 8-18 suggested a different specificity within the group of T clones mapping in the N-terminal region.

The other 6 clones mapping in the region 1-42 and responsive to BPPS1 and BBS1, did not recognized the above synthetic peptides. The five clones mapping within the C-terminal 181-211 region were assayed for the ability to recognized the peptides 171-194 and 171-185 (figure 2).

Said two peptides share the first 15 amino acids, ten of which are homologous to the C-terminal sequence of the NRU fragment. As reported in Table III, 4 clones (S105, S201, T209 and T217) of 5 were stimulated by the peptide 171-194 but not by the peptide 171-185.

This pointed out that said clones mapped in a region of 24 amino acid residues (171-194) and that their epitope was located in the second half of the region 171-194.

The clone T227 did not show any proliferation in presence of said peptides.

The clones T216 and S223, which mapped within the C-terminal 212-235 region, did not recognize the peptide 212-227.

TABLE III

| Proliferation of the T clones induced by synthetic peptides homologous to S1 | | | | | |
|---|---|---|---|---|---|
| Clones mapping within the N-terminal end | | | | | |
| Clones | Peptides (ug/ml) | | | | |
| | none | 2-15 | 6-17 | 8-18 | 27-39 |
| | | (10) | (10) | (10) | (10) |
| RR-S229 | 1.6±0.3[a] | 1.4±0.2 | 1.0±0.2 | 1.6±0.1 | 0.7±0.1 |
| RR-S232 | 0.8±0.2 | ND | 0.9±0.1 | ND | 9.2±1.1 |
| RR-T215 | 1.8±0.4 | 1.5±0.1 | 1.3±0.1 | 2.3±0.4 | 45.9±10.4 |
| RR-T219 | 1.4±0.2 | 1.1±0.1 | 1.3±0.8 | 2.0±0.9 | 2.4±2.6 |
| RR-T226 | 0.9±0.2 | ND | 0.7±0.1 | ND | 44.3±4.2 |
| Clones mapping to C-terminal end | | | | | |
| Clones | Peptides (ug/ml) | | | | |
| | none | 171-194 | 171-185 | 212-227 | |
| | | (1) | (10) | (10) | |
| RR-S105 | 1.7±0.2[a] | 96.5±12.3 | 1.4-0.1 | ND | |
| RR-S201 | 2.6±0.3 | 20.4±2.6 | 2.2±0.1 | ND | |
| RR-T209 | 1.5±0.2 | 72.5±7.8 | 1.0±0.1 | ND | |
| RR-T217 | 3.5±0.9 | 47.5±5.0 | 2.6±0.1 | ND | |
| RR-T227 | 2.5±0.3 | 2.2±0.1 | 2.4±0.1 | ND | |
| RR-T216 | 0.5±0.1 | ND | ND | 0.6±0.1 | |
| RR-S223 | 0.6±0.1 | ND | ND | 0.7±0.1 | |

a = cpm average (x $10^{-3}$)± SD of three cultures
ND = not determined
The underlined values are statistically relevant (p≤0.05)

**Claims**

1. Synthetic peptides capable of stimulating a T-cellular immunity against pertussis toxin wherein said peptides have an amino acid sequence comprising the amino acid residues of the region 1-42, 181-211 or 212-235 of the S1 subunit of pertussis toxin from Bordetella pertussis.

2. Synthetic peptides according to claim 1, wherein the amino acid sequence corresponds to the sequence of the T-epitopes comprised in the regions 1-42, 181-211 and 212-235 of the S1 subunit of the pertussis toxin from Bordetella pertussis.

3. Synthetic peptides according to claims 1 and 2, wherein said peptides are defined by the following amino acid sequences:
DDPPATVYRY DSRPPEDVFQ NGFTAWGNND NVLDHLTGRSCQ; RSVASIVGT LVRMAPVIGA CMARQAESSEA; MAAWSERAG EAMVLVYYES IAYSF; GNNDNVLDHLTGR; TRANPNPYTS RRSVA SIVGTLVRM.

4. Immunogenic anti-pertussis stimulant, comprising a synthetic peptide according to claims 1-3 combined with the synthetic peptides corresponding to the protective immunodominant B-epitope of the S1 subunit of pertussis toxin from Bordetella pertussis.

5. Acellular anti-pertussis vaccines comprising an immunologically effective amount of the peptides according to claims 1-3 or a stimulant according to claim 4.

6. Method for eliciting immunisation against pertussis comprising administering an immunologically effective amount of the vaccine according to claim 5.

7. Use of the peptides according to claims 1-3 as carriers for poorly immunogenic haptens isolated from pathogenic microorganisms.

Fig. 1

EP 0 398 322 A1

```
1                                                                    50
BPSI  [DDPPATVYRY   DSRPPEDVFQ   NGFTAWGNND   NVLDHLTGRS   CQVGSSNSAF]
BPPSI                                                     E
BBSI     2 _____ 15              27 _____ E _____ 39
            6 _____ 17
              8 _____ 18

51                                                                  100
      VSTSSSRRYT   EVYLEHRMQE   AVEAERAGRG   TGHFIGYIYE   VRADNNFYGA
                                                                 I

101                                                                 150
      ASSYFEYVDT   YGDNAGRILA   GALATYQSEY   LAHRRIPPEN   IRRVTRVHN
                                                                 T

151                                                                 200
      GITGETTTTE   YSNARYVSQQ   TRANPNPYTS   [RRSVASIVGT   LVRMAPVIGA]
                   P L                       T            T          T
                   P                                                 T
                                    171 _____ 194
                                    171 _____ 185

201                                                    235
      [CMARQAESSE  A]  [MAAWSERAG   EAMVLVYYES   IAYSF]
                 P              T
                 P
              212 _____ 227
```

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | EP-A-0 266 325 (TRION FORSKIN)<br>* Completely *<br>--- | 1-4 | C 07 K 7/08<br>C 07 K 7/10<br>A 61 K 37/02<br>A 61 K 39/00 |
| X | EP-A-0 279 151 (TRION FORSKIN)<br>* Completely *<br>--- | 1-4 | |
| P,Y | EP-A-0 320 866 (SCLAVO)<br>* Completely *<br>--- | 1-4 | |
| P,X | PROC. NATL. ACAD. SCI. USA, vol. 87, 1990, pages 1347-1351; P. ASKELÖF et al.: "Protective immunogenicity of two synthetic peptides selected from the amino acid sequence of Bordetella pertussis toxin subunit S1"<br>* Complete article *<br>--- | 1-4 | |
| P,A | CHEMICAL ABSTRACTS, vol. 110, 1989, page 603, abstract no. 171325d, Columbus, Ohio, US; K.J. KIM et al.: "Epitopes on the S1 subunit of pertussis toxin recognized by monoclonal antibodies", & INFECT. IMMUN. 1989, 57(3), 944-50<br>* Abstract *<br>--- | 3-4 | TECHNICAL FIELDS SEARCHED (Int. Cl.5)<br><br>C 07 K<br>A 61 K |
| P,X | J. EXP. MED., vol. 169, May 1989, pages 1519-1532, The Rockefeller University Press; M. TERESA DE MAGISTRIS et al.: "Human T cell clones define S1 subunit as the most immunogenic moiety of pertussis toxin and determine its epitope map"<br>* Complete article *<br>----- | 1-7 | |

The present search report has been drawn up for all claims

| Place of search | Date of completion of the search | Examiner |
|---|---|---|
| THE HAGUE | 21-08-1990 | RAJIC M. |